**Europäisches Patentamt**

**European Patent Office**

**Office européen des brevets**

(19)

(11) Numéro de publication : **0 275 232 B1**

(12)

# FASCICULE DE BREVET EUROPEEN

(45) Date de publication du fascicule du brevet :
**12.08.92 Bulletin 92/33**

(51) Int. Cl.⁵ : **A61M 21/00**

(21) Numéro de dépôt : **88810003.9**

(22) Date de dépôt : **06.01.88**

(54) **Appareil de rélaxation et de stimulation psychosensorielle.**

(30) Priorité : **16.01.87 CH 159/87**

(43) Date de publication de la demande :
**20.07.88 Bulletin 88/29**

(45) Mention de la délivrance du brevet :
**12.08.92 Bulletin 92/33**

(84) Etats contractants désignés :
**AT BE CH DE GR LI LU NL SE**

(56) Documents cités :
**BE-A- 903 721**
**BE-A- 904 633**
**BE-A- 904 731**
**FR-A- 1 161 427**
**US-A- 3 826 250**

(73) Titulaire : **NEW SPACE S.A.**
**Rue de Verdeaux 4**
**CH-1020 Renens (CH)**

(72) Inventeur : **Soder, Hugo**
**Cotterd 4 Territet**
**CH-1820 Montreux (CH)**

(74) Mandataire : **Hranitzky, Wilhelm Max et al**
**c/o WILLIAM BLANC & CIE Conseils en**
**Propriété Industrielle SA 6, rue de la Grotte**
**CH-1003 Lausanne (CH)**

**Description**

La présente invention a pour objet un appareil de relaxation et de stimulation psychosensorielle.

L'homme voit aujourd'hui sa santé de plus en plus menacée par son propre comportement, par les exigences socioprofessionnelles de la vie quotidienne et les difficultés qu'il a à s'y adapter. Le stress est en passe de devenir une des causes les plus importantes des maladies de notre siècle.

La stimulation psychosensorielle consiste en la synthèse de plusieurs psychotechniques de pointe, telles la sophrologie, la suggestopédie, l'hypnopédie, l'information subliminale, etc., dont le point commun se trouve dans la nécessité d'une base de relaxation optimale. La stimulation psychosensorielle applique le principe connu de l'isolation sensorielle, qui permet, en supprimant les causes extérieures de stress grâce au contrôle total d'un environnement déterminé, d'atteindre des états de relaxation d'une profondeur inhabituelle. En se plaçant simplement dans une enceinte d'isolation sensorielle, le sujet se trouve immédiatement soulagé de l'effort permanent de maintien de son équilibre homéostatique. La relaxation totale qu'il obtient ainsi favorise l'émergence d'états de conscience caractérisés par une focalisation de l'esprit sur lui-même. Il péut alors consacrer son énergie à l'exploration et au développement de son espace mental, soit de manière active (méditation créatrice, recherche, idéation), soit de manière passive (contemplation, rêve éveillé). Dans une perspective plus simple de détente et de repos, l'isolation sensorielle est d'une efficacité rare. En effet, il est généralement admis qu'une séance d'une heure équivaut en relaxation mentale à quatre heures de sommeil profond.

Les premières expériences d'isolation sensorielle eurent lieu aux Etats-Unis, il y a une trentaine d'années. Le sujet flottait sur de l'eau saturée de sel, contenue dans un caisson isolé phoniquement et étanche à la lumière. Puis, on étudia une méthode plus simple et surtout plus hygiénique dans laquelle l'eau salée était remplacée par un matelas spécial. La technique utilisant l'eau salée présente en effet de nombreux inconvénients qui furent rapidement mis en évidence par l'usage public. Outre les aspects psychologiques (phobie de l'eau, etc.), l'humidité dégagée, le sel agressif pour le corps, et la consistance visqueuse de la solution nuisent au confort d'utilisation, donc au rendement de l'appareil. De plus, l'hygiène de ce système est aléatoire du fait de l'impossibilité de changer l'eau entre chaque séance. Les progrès de la technologie dans le domaine des matières synthétiques permettent actuellement la construction d'un support sec à comportement fluide présentant les mêmes avantages que le flottement sur de l'eau salée et un confort quasi optimal Voir document US-A-3 826 250.

Le but de la présente invention est de proposer un appareil de relaxation et de stimulation psychosensorielle conçu sur un principe de modules polyvalents et évolutifs, facilement transformables pour pouvoir accueillir des équipements supplémentaires ou personnalisés et qui permettront un développement sans contraintes des multiples possibilités d'application de ce type d'appareil.

A cet effet, l'invention concerne un appareil de relaxation et de stimulation psychosensorielle, caractérisé en ce qu'il comporte une enceinte destinée à créer un espace protégé et isolé de son environnement dans lequel prend place l'utilisateur, relié à une armoire de commande comprenant un ordinateur, un support d'informations et son lecteur, un appareil de conditionnement de l'air, un ionisateur et un diffuseur d'arômes, l'enceinte étant munie d'un sas à assistance pneumatique et comportant une couchette à comportement fluide et une pluralité d'appareils permettant la génération de stimulations psychosensorielles, l'enceinte comprenant une enveloppe extérieure étanche aux radiations et aux ondes électromagnétiques, réalisée sous la forme d'une coque du type sandwich comportant une matière d'isolation thermique et phonique prise entre deux parois en matière synthétique comportant une matière non toxique ayant un effet d'écran aux radiations, cette matière étant adjointe à la matière synthétique lors de la fabrication des parois, et un écran métallique destiné à créer un effet de cage de Faraday.

Selon une forme d'exécution, les deux parois de la coque sont des parois composites en fibres de verre et résine synthétique.

L'écran peut être noyé dans une des parois de la coque ou placé entre les parois de la coque.

Dans une forme d'exécution la couchette est chauffante et est contenue dans un bac synthétique à haut pouvoir d'isolation thermique, le fond du bac comportant au moins une couche de cylindres en matière synthétique élastique ou en caoutchouc gonflés d'air, la pression de chaque cylindre étant réglable séparément, le fond du bac pouvant comporter une pluralité de couches de cylindres gonflés d'air, disposés horizontalement dans le sens de la largeur et recouverts sur toute la surface de la couchette par une plaque de chauffage souple et imperméable contenant des résistances électriques et sur laquelle est disposée au moins un élément creux en matière synthétique élastique ou en caoutchouc déformable et contenant un liquide ou un colloïde, ledit élément étant fixé sur un treillis élastique destiné à assurer sa position, l'ensemble pouvant être recouvert d'un rembourrage de mousse ou d'ouate synthétique permettant une circulation de l'air, et d'une housse.

Selon une variante d'exécution, le bac comporte une pluralité de sphères creuses en matière synthétique élastique ou en caoutchouc, disposées côte-à-côte sur la plaque de chauffage et fixées sur le treillis élastique.

L'enceinte peut comporter un dispositif d'éclairage intérieur comprenant un faisceau de fibres optiques réunies à l'une de leurs extrémités à une source lumineuse et dont les secondes extrémités affleurent à la surface du revêtement intérieur de l'enceinte.

Selon une forme d'exécution du dispositif d'éclairage, les secondes extrémités des fibres optiques sont réparties et noyées dans une plaque de résine synthétique opaque dont la face visible est polie, leurs sections d'extrémité affleurant à la surface de la face visible de la plaque de résine synthétique.

L'appareil peut comporter en outre une pluralité de filtres couleurs mobiles, destinés à coopérer avec le dispositif d'éclairage et commandeés par un moteur.

Le programme de stimulation psychosensorielle comporte par exemple une étape d'induction modificatrice du niveau de conscience de l'utilisateur destiné à le conduire à un état de profonde relaxation, une étape d'intériorisation dans laquelle l'utilisateur vit en toute lucidité des états de conscience modifiés et une étape de réintégration destinée à la réapparition de la conscience corporelle et de la conscience de veille de l'utilisateur, synchronisée avec la réapparition progressive de l'environnement de base dans l'enceinte.

D'autres caractéristiques et avantages de l'invention ressortiront mieux de la description qui suit, donnée à titre d'exemple.

L'appareil comprend deux éléments principaux : une enceinte destinée à créer un espace protégé et isolé de son environnement dans lequel prend place l'utilisateur et une armoire de commande, reliée à l'enceinte.

L'armoire de commande comprend un ordinateur qui gère l'ensemble des opérations se déroulant dans l'enceinte. Un lecteur de bandes magnétiques sert à transmettre les informations et les effets sons et images, en fonction des programmes et de codes enregistrés sur des bandes magnétiques selon une méthode appropriée. L'armoire comporte en outre un appareillage de conditionnement de l'air dans l'enceinte, un ionisateur et un diffuseur d'arômes. Le lecteur de bandes magnétiques peut être remplacé par tout lecteur d'informations numérique ou analogique, tel que par exemple un lecteur de disques laser ou de vidéodisques.

L'enceinte est constituée d'une enveloppe extérieure, ou coque, étanche aux radiations et aux ondes électromagnétiques. Cette coque est du type sandwich, composée de laine minérale ou de toute autre matière d'isolation thermique et phonique, placée entre deux parois composites en fibre de verre et résine de polyesther ou toute autre matière synthétique, rendue étanche aux radiations grâce à l'adjonction de sulfate de baryum à la résine de polyesther lors de la fabrication des parois composites, ou de toute autre matière inerte non toxique ayant un effet d'écran aux radiations. Cette coque est en outre étanche aux ondes électromagnétiques par effet de cage de Faraday, grâce à un écran métallique, réalisé par exemple sous la forme d'un treillis d'acier inoxydable placé dans l'une des parois de la coque ou entre les deux parois de la coque. Dans cette enceinte sont disposés une pluralité d'appareils permettant la génération de stimulations psychosensorielles, telles que par exemple des enceintes acoustiques, un éclairage indirect, des lampes phosphènes et un projecteur holographique. Ces appareils sont utilisés en fonction des programmes et servent à créer chez l'individu un état de relaxation et de réceptivité totale. La conception intérieure est réalisée de façon à pouvoir recevoir d'autres éléments tels que par exemple un moniteur vidéo, un projecteur holographique, un ordinateur, un interphone, etc. L'enceinte comporte en outre une couchette sur laquelle prend place l'utilisateur. La couchette est chauffante et réalisée de façon à se mouler parfaitement à la surface du corps de l'utilisateur et à permettre à cet utilisateur de prendre une position de relaxation idéale.

Selon un mode d'exécution préférentiel, la couchette est contenue dans un bac synthétique rectangulaire, à haut pouvoir d'isolation thermique. Le fond de ce bac contient plusieurs couches de cylindres en caoutchouc ou en matière synthétique élastique, gonflés d'air, la pression de chaque cylindre étant réglable séparément. Ces cylindres sont disposés horizontalement, dans le sens de la largeur. Ils sont recouverts sur toute la surface de la couchette par une plaque de chauffage souple et imperméable, contenant des résistances électriques. Des sphères creuses en caoutchouc ou en matière synthétique élastique, à très fines parois, hautement déformables et contenant de l'eau ou tout autre fluide liquide ou colloïde, sont réparties côte-à-côte sur cette plaque. Ces sphères sont fixées à leur base par leur valve de remplissage, sur un treillis élastique qui assure leur position. La couchette est recouverte d'un fin rembourrage de mousse ou d'ouate synthétique laissant circuler l'air et enfin d'une housse en coton à mailles élastiques. Cette housse est surmontée d'une seconde housse interchangeable qui viendra en contact avec le corps de l'utilisateur.

L'éclairage intérieur de l'enceinte peut être réalisé avec un effet "ciel étoilé", en utilisant un faisceau de fibres optiques réunies à l'une de leurs extrémités à l'intérieur d'un cylindre métallique contenant une source lumineuse. Les secondes extrémités, libres, des fibres optiques affleurent à la surface du revêtement intérieur de l'enceinte. Ces secondes extrémités peuvent par exemple être réparties et noyées dans une plaque de résine synthétique opaque dont la face visible est polie et laisse affleurer à sa surface les sections d'extrémité des fibres optiques, qui apparaissent ainsi comme autant de petits points lumineux. Un ou plusieurs filtres colorés peuvent éventuellement être interposés ou disposés de façon à coopérer avec le dispositif d'éclairage, en étant commandé par un moteur.

Le contrôle des divers stimuli perceptibles par les sens de l'utilisateur est assuré par un microprocesseur. En modifiant les sensations de l'utilisateur par un logiciel adéquat, il est possible de recréer un environnement du type idéal, propice à l'épanouissement de la conscience et d'y introduire un programme de stimulations psychosensorielles. Le fonctionnement de l'appareil de l'invention est étudié pour un confort et une simplicité d'utilisation maximum. L'utilisateur place une cassette ou tout autre support adéquat contenant le programme choisi dans le lecteur d'informations. Il s'installe dans l'enceinte et referme le sas d'accès. Le logiciel de contrôle se met alors automatiquement en marche et donne verbalement les instructions nécessaires. Guidé par le programme, l'utilisateur est placé très rapidement dans un état de relaxation profond. Le programmes de stimulation psychosensorielle comporte une étape d'induction modificatrice du niveau de conscience de l'utilisateur destiné à le conduire à un état de profonde relaxation, de perte de notion de son corps et de focalisation de son esprit sur lui-même, une étape d'intériorisation dans laquelle l'utilisateur vit en toute lucidité des états de conscience modifiés tels que le rêve éveillé, la dépersonnalisation, la perception du temps à la vitesse de la pensée, la conscience olistique, l'hypersensibilité psychique à des manifestations parapsychologiques. A ce moment, toutes les conditions nécessaires à une exploration de l'espace intérieur sont réunies et le programme poursuit la conduite de la séance en fonction de son but spécifique. Pour terminer, l'utilisateur est ramené progressivement à un niveau de conscience compatible avec l'extérieur et il peut quitter l'enceinte sans effort de réadaptation. Pendant toute la séance, le microprocesseur affiche sur un moniteur vidéo extérieur les différentes phases du programme en cours, ceci pour permettre un contrôle visuel externe.

L'appareil de l'invention offre un vaste choix d'applications aux niveaux physiques, psychiques et mentaux :

– préservation de la santé (contrôle du poids, cesser de fumer, retrouver le sommeil, prévention des maladies cardio-vasculaires),

– développement du psychisme (pensée positive, confiance en soi, lutte contre le trac et contre la timidité, créativité),

– développement du mental (apprentissage de langues, mémorisation, raisonnements abstraits),

– applications médicales (troubles psychiques, allergies, intoxications chroniques),

– traitement des maladies psychosomatiques (asthme, ulcères, hypertension),

– applications spécifiques (entraînement sportif, préparation à l'accouchement, harmonisation sexuelle, préparation d'examens),

– récupération du décalage horaire (régulation du sommeil et reprise du rythme biologique normal).

Par la polyvalence de leurs applications, les appareils de relaxation et de stimulation psychosensorielle se présentent comme de véritables véhicules d'exploration de nouveaux états de relaxation et de conscience. C'est pourquoi on leur donne généralement le nom de navettes.

## Revendications

1. Appareil de relaxation et de stimulation psychosensorielle, comportant une enceinte destinée à créer un espace protégé et isolé de son environnement dans lequel prend place l'utilisateur, relié à une armoire de commande comprenant un ordinateur, un support d'informations et son lecteur, un appareil de conditionnement de l'air, un ionisateur et un diffuseur d'arômes, caractérisé en ce qu'il comporte l'enceinte munie d'un sas à assistance pneumatique et comportant une couchette à comportement fluide et une pluralité d'appareils permettant la génération de stimulations psychosensorielles, l'enceinte comprenant une enveloppe extérieure étanche aux radiations et aux ondes électromagnétiques, réalisée sous la forme d'une coque du type sandwich comportant une matière d'isolation thermique et phonique prise entre deux parois en matière synthétique comportant une matière non toxique ayant un effet d'écran aux radiations, cette matière étant adjointe à la matière synthétique lors de la fabrication des parois, et un écran métallique destiné à créer un effet de cage de Faraday.

2. Appareil selon la revendication 1, caractérisé en ce que les deux parois de la coque sont des parois composites en fibres de verre et résine synthétique.

3. Appareil selon l'une des revendications 1 ou 2, caractérisé en ce que l'écran est noyé dans une des parois de la coque.

4. Appareil selon l'une des revendications 1 ou 2, caractérisé en ce que l'écran est placé entre les parois de la coque.

5. Appareil selon l'une des revendications précédentes, caractérisé en ce que la couchette est chauffante et est contenue dans un bac synthétique à haut pouvoir d'isolation thermique, le fond du bac comportant au moins une couche de cylindres en matière synthétique élastique ou en caoutchouc gonflés d'air, la pression de chaque cylindre étant réglable séparément.

6. Appareil selon la revendication 5, caractérisé en ce que le fond du bac comporte une pluralité de couches de cylindres gonflés d'air, disposés horizontalement dans le sens de la largeur et recouverts sur toute la surface de la couchette par une plaque de chauffage souple et imperméable contenant des résistances électriques et sur laquelle est disposée au moins un élément creux en matière synthétique élastique ou en caoutchouc déformable et contenant un liquide ou un colloïde, ledit élément étant fixé sur un treillis élastique destiné à assurer sa position, l'ensemble étant en outre recouvert d'un rembourrage de mousse ou d'ouate synthétique permettant une circulation de l'air, et d'une housse.

7. Appareil selon la revendication 6, caractérisé en ce qu'il comporte une pluralité de sphères creuses en matière synthétique élastique ou en caoutchouc, disposées côte-à-côte sur la plaque de chauffage et fixées sur le treillis élastique.

8. Appareil selon l'une des revendications précédentes, caractérisé en que l'enceinte comporte un dispositif d'éclairage intérieur comprenant un faisceau de fibres optiques réunies à l'une de leurs extrémités à une source lumineuse et dont les secondes extrémités affleurent à la surface du revêtement intérieur de l'enceinte.

9. Appareil selon la revendication 8, caractérisé en ce que les secondes extrémités des fibres optiques sont réparties et noyées dans une plaque de résine synthétique opaque dont la face visible est polie, leurs sections d'extrémité affleurant à la surface de la face visible de la plaque de résine synthétique.

10. Appareil selon l'une des revendications précédentes, caractérisé en ce qu'il comporte une pluralité de filtres de couleurs mobiles, destinés à coopérer avec le dispositif d'éclairage et commandés par un moteur.

11. Appareil selon l'une des revendications précédentes, caractérisé en ce que le programme de stimulation psychosensorielle comporte une étape d'induction modificatrice du niveau de conscience de l'utilisateur destinée à le conduire à un état de profonde relaxation, une étape d'intériorisation dans laquelle l'utilisateur vit en toute lucidité des états de conscience modifiés et une étape de réintégration destinée à la réapparition de la conscience corporelle et de la conscience de veille de l'utilisateur, synchronisée avec la réapparition progressive de l'environnement de base dans l'enceinte.

**Patentansprüche**

1. Gerät zur Entspannung und zur psychosonrischen Stimulation, welches ein Gehäuse zur Erzeugung eines geschützten und von seiner Umgebung isolierten Raumes aufweist, in dem der Benutzer Platz nimmt, und welcher mit einem Steuerschrank verbunden ist, der einen Computer, einen Informationsträger und dessen Lesevorrichtung, ein Klimatisierungsgerät, einen Ionisator und einen Aromaverteiler aufweist, dadurch gekennzeichnet, dass es ein Gehäuse aufweist, welches mit einer pneumatisch unterstützten Schleusenkammer versehen ist und eine Lagerstätte mit fliessendem Verhalten sowie eine Mehrzahl von Geräten aufweist, welche die Erzeugung von psychosensorischen Stimulationen erlauben, wobei das Gehäuse eine äussere, gegenüber Strahlungen und elektromagnetischen Wellen undurchlässige Hülle aufweist, die in der Form einer sandwichartigen Schale verwirklicht ist, die ein thermisch und akustisch isolierendes Material enthält, das zwischen zwei Wänden aus synthetischem Material eingeschlossen ist, welches ein ungiftiges, eine abschirmende Wirkung gegenüber Strahlungen besitzendes Material enthält, wobei dieses Material dem synthetischen Material anlässlich der Fertigung der Wände beigefügt wird, sowie eine metallische Abschirmung zur Erzeugung eines Faraday'schen Käfig-Effekts enthält.

2. Gerät nach Patentanspruch 1, dadurch gekennzeichnet , dass die beiden Wände der Schale Kompositwände aus Glasfasern und Kunstharz sind.

3. Gerät nach einem der Patentansprüche 1 oder 2, dadurch gekennzeichnet, dass die Abschirmung in einer der Wände der Schale eingegossen ist.

4. Gerät nach einem der Patentansprüche 1 oder 2, dadurch gekennzeichnet, dass die Abschirmung zwischen den Wänden der Schale angeordnet ist.

5. Gerät nach einem der vorhergehenden Patentansprüche, dadurch gekennzeichnet, dass die Lagerstätte heizbar ist und in einem synthetischen Becken mit hoher thermischer Isolierfähigkeit aufgenommen ist, wobei der Boden des Beckens mindestens eine Schicht von Zylindern aus elastischem synthetischem Material oder aus Kautschuk aufweist, die mit Luft aufgeblasen sind, wobei der Druck jedes Zylinders getrennt regulierbar ist.

6. Gerät nach Patentanspruch 5, dadurch gekennzeichnet, dass der Boden des Beckens eine Mehrzahl von Schichten von mit Luft aufgeblasenen Zylindern aufweist, welche horizontal in der Richtung der Breite angeordnet sind und auf der ganzen Oberfläche der Lagerstätte von einer elastischen und undurchlässigen Heizplatte bedeckt sind, welche elektrische Widerstände enthält und auf der mindestens ein hohles Element aus elastischem Kunststoff oder aus verformbarem Kautschuk angeordnet ist, welches eine Flüssigkeit oder ein Kolloid enthält, wobei dieses Element auf einem elastischen Gitter befestigt ist, das zur Sicherung von dessen Lage dient, wobei das Ganze ferner mit einer Polsterung aus synthetischem Schaum oder Watte, die eine Luftzirkulation erlaubt, sowie mit einem Überzug bedeckt ist.

7. Gerät nach Patentanspruch 6, dadurch gekennzeichnet, dass es eine Mehrzahl von Hohlkugeln aus elastischem Kunststoff oder aus Kautschuk aufweist, welche nebeneinander auf der Heizplatte angeordnet sind und auf dem elastischen Gitter befestigt sind.

8. Gerät nach einem der vorhergehenden Patentansprüche, dadurch gekennzeichnet, dass das Gehäuse eine Innenbeleuchtungs-Vorrichtung aufweist, welche ein Bündel von optischen Fasern enthält, die an einem ihrer Enden mit einer Lichtquelle vereinigt sind und deren zweite Enden auf der Höhe der Oberfläche der Innenauskleidung des Gehäuses liegen.

9. Gerät nach Patentanspruch 8, dadurch gekennzeichnet, dass die zweiten Enden der optischen Fasern in einer Platte aus lichtundurchlässigem Kunstharz, deren sichtbare Fläche poliert ist, verteilt und eingebettet sind, wobei die Querschnitte der Enden in der Oberfläche der sichtbaren Fläche der Kunstharz-Platte liegen.

10. Gerät nach einem der vorhergehenden Patentansprüche, dadurch gekennzeichnet, dass es eine Mehrzahl von beweglichen Farbfiltern aufweist, die zur Zusammenwirkung mit der Beleuchtungsvorrichtung bestimmt sind und von einem Motor gesteuert werden.

11. Gerät nach einem der vorhergehenden Patentansprüche, dadurch gekennzeichnet, dass das Programm der psychosensorischen Stimulation eine Phase der Induzierung einer Veränderung des Bewusstseinsniveaus des Benutzers enthält, welche dazu bestimmt ist, diesen in einen Zustand tiefer Entspannung zu bringen, eine Phase der Verinnerlichung, in welcher der Benutzer mit völliger Klarheit veränderte Bewusstseinszustände erlebt, und eine Phase der Wiedereinsetzung, welche zum Wiedereintreten des körperlichen Bewusstseins und des Wachheitsbewusstseins des Benutzers dient, welche mit dem progressiven Wiedererscheinen der Basisumgebung im Gehäuse synchronisiert ist.

## Claims

1. An apparatus for relaxation and psychosensorial stimulation comprising an enclosure designed for creating a space, protected and isolated from its environment, into which the user places himself, connected to a control box comprising a computer, an information support and its reader, an air conditioner, an ionizer and a fragrance diffuser, characterized in that it comprises an enclosure provided with a pneumatically assisted lock chamber and comprising a berth with a fluid behaviour and a plurality of apparatuses capable of generating psychosensorial stimulations, the enclosure comprising an outer casing impervious to radiations and to electromagnetic waves, constructed in the form of a sandwich-type shell comprising a thermally and acoustically insulating material trapped between two walls of a synthetic material comprising a non-toxic material acting as a shield to radiations, this material being adjoined to the synthetic material during the manufacture of the walls, and a metallic shield designed to create a Faraday cage effect.

2. An apparatus according to claim 1, characterized in that the two walls of the shell are composite walls of glass fibers and of synthetic resin.

3. An apparatus according to one of claims 1 or 2, characterized in that the shield is embedded in one of the shell walls.

4. An apparatus according to one of claims 1 or 2, characterized in that the shield is placed between the shell walls.

5. An apparatus according to one of the preceding claims, characterized in that the berth is a heating berth and is received within a synthetic container having a high thermal insulating capacity, the bottom of the container comprising at least one layer of air inflated cylinders of a synthetic elastic material or of rubber, the pressure of each cylinder being adjustable separately.

6. An apparatus according to claim 5, characterized in that the bottom of the container comprises a plurality of layers of air inflated cylinders, positioned horizontally in the widthwise direction and recovered on the whole surface of the berth with a pliable impervious heating plate containing electric resistors and on which is laid at least one hollow element of a synthetic elastic material or of flexible rubber, and containing a liquid or a colloid, said element being attached to an elastic lattice designed for maintaining its position, the assembly being further recovered with a padding of cellular material or of synthetic wool allowing an air circulation, and a covering.

7. An apparatus according to claim 6, characterized in that it comprises a plurality of hollow spheres of synthetic elastic material or of rubber, laid side by side on the heating plate and fixed to the elastic framework.

8. An apparatus according to one of the preceding claims, characterized in that the enclosure includes an internal lighting device comprising a bunch of optical fibers, connected at one of their end to a light source and the second ends of which are flush with the surface of the inner lining of the enclosure.

9. An apparatus according to claim 8, characterized in that the second ends of the optical fibers are spread out and embedded in a plate of an opaque synthetic resin the outer visible surface of which is polished, their end sections being flush with the surface of the visible face of the plate of synthetic resin.

10. An apparatus according to one of the preceding claims, characterized in that it comprises a plurality of movable color filters, designed for cooperating with the lightening device and controlled by a motor.

11. An apparatus according to one of the preceding claims, characterized in that the psychosensorial stimulation programme comprises an induction step modifying the level of consciousness of the user, designed to induce him into a state of deep relaxation, a step of interiorization during which the user experiences, while remaining fully lucid, states of modified consciousness and a step of reintegration deisgned for restoring the body consciousness and the waking state consciousness of the user, synchronized with the progressive reappearing of the basic environment in the enclosure.